# EUROPEAN PATENT APPLICATION

(11) **EP 4 654 210 A1**
(43) Date of publication of application: **26.11.2025**
(21) Application number: 24760335.0
(22) Date of filing: 20.02.2024
(51) Int. Cl.: G16H 20/17, A61M 5/142

(54) **COMPUTER PROGRAM, MEDICAL PUMP SETTING DEVICE, MEDICAL PUMP SETTING SYSTEM, AND MEDICAL PUMP SETTING METHOD**

(30) Priority: 24.02.2023 JP 2023027493
(71) Applicant: Terumo Kabushiki Kaisha, Tokyo 151-0072 (JP)
(72) Inventor: KAZAMA, Shigeyuki, Ashigarakami-gun, Kanagawa 259-0151 (JP); HARA, Yuki, Ashigarakami-gun, Kanagawa 259-0151 (JP); ASAMA, Koichiro, Ashigarakami-gun, Kanagawa 259-0151 (JP)
(74) Representative: KIPA AB
(86) International application number: PCT/JP2024/005912
(87) International publication number: WO 2024/177037

(57) **Abstract**

Provided are a computer program, a medical pump setting device, a medical pump setting system, and a medical pump setting method capable of easily setting a cooperative administration function of a medical pump.

The computer program causes a computer to execute processing of: displaying a first cooperative administration screen to receive a setting parameter for setting a start time of cooperative administration for preventing a remaining infusion amount during a cooperative administration operation of causing a plurality of medical pumps to cooperate from being equal to or smaller than an alarm value; and displaying the start time when the setting parameter is input or selected on the first cooperative administration screen.

## Description

### Technical Field

The present invention relates to a computer program, a medical pump setting device, a medical pump setting system, and a medical pump setting method.

### Background Art

A medical pump such as an infusion pump or a syringe pump is a medical device for administering various combinations of drugs necessary for treatment through an indwelling needle puncturing a vein and an infusion line in order to maintain a body fluid of a patient during treatment or recuperation in a normal state. There is an upper limit to the amount of medicine that can be administered by one medical pump. Therefore, in order to continuously administer a large amount of the same type of drug to a patient, it is necessary to prepare the number of medical pumps corresponding to an amount to be administered and to continuously operate a plurality of medical pumps. For this reason, there is a medical pump having a cooperative administration function.

In such a medical pump, a drug library in which information, such as a flow rate of a drug and a limit value of the flow rate, is defined for each type of drug is stored (see Patent Literature 1), and it is necessary to create a drug library in which setting items for the cooperative administration function are set in advance in order to use the cooperative administration function.

### Citation List

### Patent Literature

Patent Literature 1: JP 2015-91280 A

### Summary of Invention

### Technical Problem

However, in many cases, there are many setting items for the cooperative administration function, and it is not clear how to set the setting items. In particular, it is necessary to calculate, from the many setting items, a start time for determining when to start cooperation so as to prevent a remaining amount of a drug of a preceding medical pump does not fall below an alarm issuing remaining amount at the cooperation end.

The present invention has been made in view of such circumstances, and an object thereof is to provide a computer program, a medical pump setting device, a medical pump setting system, and a medical pump setting method which are capable of easily setting a cooperative administration function of a medical pump.

### Solution to Problem

(1) A computer program according to the present invention causes a computer to execute processing of: displaying a first cooperative administration screen to receive setting parameters for setting a start time of cooperative administration for preventing a remaining infusion amount during a cooperative administration operation of causing a plurality of medical pumps to cooperate from being equal to or smaller than an alarm value; and displaying the start time when the setting parameters are input or selected on the first cooperative administration screen.
   According to embodiments of the present invention, the following are provided.
(2) The computer program according to (1), for causing the computer to execute processing of displaying a second cooperative administration screen including a number of cooperation steps, a cooperation step time, and a flow rate in each of the cooperation steps of each of the plurality of medical pumps when the setting parameters are input or selected on the first cooperative administration screen.
(3) The computer program according to (1) or (2), for causing the computer to execute processing of displaying a second cooperative administration screen including a cooperation image indicating transition of a flow rate of each of the plurality of medical pumps when the setting parameters are input or selected on the first cooperative administration screen.
(4) The computer program according to any one of (1) to (3), for causing the computer to execute processing of: receiving an operation on the cooperation image indicating the transition of the flow rate of each of the plurality of medical pumps in the second cooperative administration screen; and adjusting and displaying at least one of a cooperation step time and the flow rate in each cooperation step of each of the plurality of medical pumps displayed on the first cooperative administration screen in accordance with the received operation.
(5) In the computer program according to any one of (1) to (4), the plurality of medical pumps include a preceding medical pump that gradually decreases a flow rate during the cooperative administration operation, and the start time is a period of time required when an amount to be fed from the preceding medical pump in the cooperative administration is fed at a set flow rate of the preceding medical pump.
(6) In the computer program according to any one of (1) to (5), the plurality of medical pumps include a preceding medical pump that gradually decreases a flow rate during the cooperative administration operation, and the setting parameters include a syringe size of the preceding medical pump, a set flow rate, a remaining amount at cooperation completion, a number of cooperation steps, and a cooperation time.
(7) A medical pump setting device according to the present invention includes a control unit, the control unit displaying a first cooperative administration screen to receive a setting parameter for setting a start time of cooperative administration for preventing a remaining infusion amount during a cooperative administration operation of causing a plurality of medical pumps to cooperate from being equal to or smaller than an alarm value, and displaying the start time when the setting parameter is input or selected on the first cooperative administration screen.
(8) A medical pump setting system according to the present invention includes: the above-described medical pump setting device; and a plurality of medical pumps.
(9) A medical pump setting method according to the present invention including: displaying a first cooperative administration screen to receive a setting parameter for setting a start time of cooperative administration for preventing a remaining infusion amount during a cooperative administration operation of causing a plurality of medical pumps to cooperate from being equal to or smaller than an alarm value; and displaying the start time when the setting parameter is input or selected on the first cooperative administration screen.

### Advantageous Effects of Invention

According to the present invention, the cooperative administration function of the medical pump can be easily set.

### Brief Description of Drawings

Fig. 1 is a diagram illustrating an example of a configuration of a medical pump setting system of the present embodiment.
Fig. 2 is a view illustrating an example of a configuration of a syringe pump.
Fig. 3 is a view illustrating an example of a cooperative administration operation of syringe pumps.
Fig. 4 is a view illustrating an example of a state of a preceding syringe pump during the cooperative administration operation.
Fig. 5 is a view illustrating an example of a case where a control unit calculates a start time.
Fig. 6 is a view illustrating an example of a pump basic setting screen.
Fig. 7 is a view illustrating a first example of a cooperative administration simple setting screen.
Fig. 8 is a view illustrating a first example of a cooperative administration screen.
Fig. 9 is a view illustrating a second example of the cooperative administration screen.
Fig. 10 is a view illustrating a third example of the cooperative administration screen.
Fig. 11 is a view illustrating a second example of the cooperative administration simple setting screen.
Fig. 12 is a view illustrating an example of a processing procedure of a medical pump setting device.

### Description of Embodiments

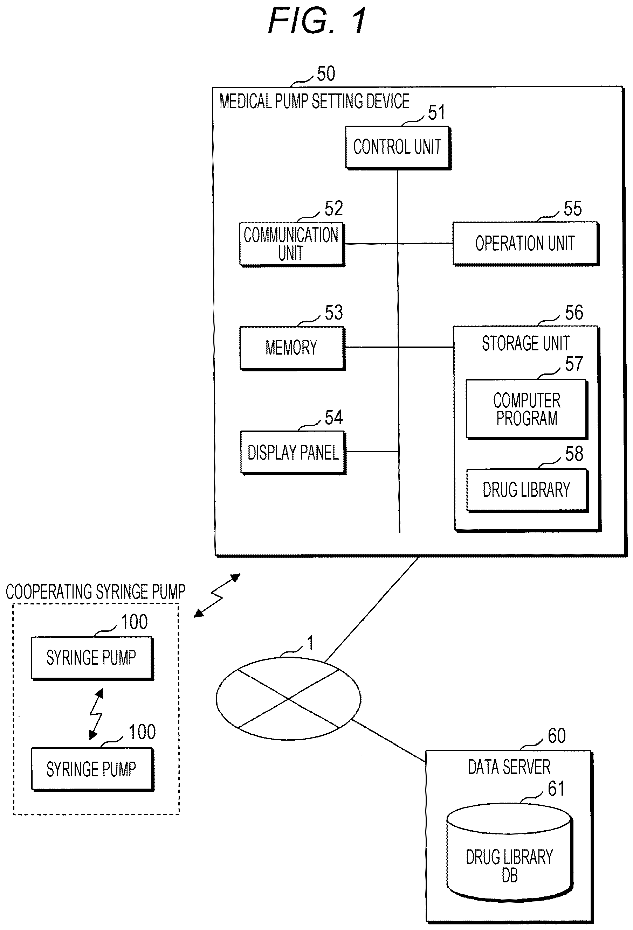

Hereinafter, an embodiment of the present invention will be described. Fig. 1 is a diagram illustrating an example of a configuration of a medical pump setting system of the present embodiment. The medical pump setting system of the present embodiment includes a medical pump setting device 50 and a plurality of syringe pumps 100 as medical pumps. Although a configuration in which a syringe pump that injects a relatively small amount of a drug is used as a medical pump will be described in the present specification, the medical pump is not limited to the syringe pump, and may be an infusion pump that injects a relatively large amount of a drug. The medical pump setting device 50 is connected to the plurality of (two in the example of Fig. 1) syringe pumps 100 via various communication networks such as serial communication, Bluetooth (registered trademark), near field communication (NFC), a wired LAN, and a wireless LAN. Each of the two syringe pumps 100 has a cooperative administration function and constitute one cooperating syringe pump. The two syringe pumps 100 perform a cooperative administration operation. The number of cooperating syringe pumps is not limited to one. The medical pump setting system may include a data server 60 having a drug library DB 61. The medical pump setting device 50 is connected to the data server 60 via a communication network 1. Each of the syringe pumps 100 is connected to an in-hospital IT system (not illustrated) via an in-hospital communication system. Examples of the in-hospital IT system include a hospital information system such as an electronic medical record, an assistance system that assists examination and treatment, and surgical and anesthesia systems.

The medical pump setting device 50 includes a control unit 51 that controls the entire device, a communication unit 52, a memory 53, a display panel 54, an operation unit 55, and a storage unit 56. The medical pump setting device 50 can include, for example, a desktop or notebook personal computer, but may include a tablet terminal. The medical pump setting device 50 has a drug library editing function.

The control unit 51 may be configured by incorporating a required number of central processing units (CPUs), micro-processing units (MPUs), graphics processing units (GPUs), and the like. In addition, the control unit 51 may be configured by combining digital signal processors (DSPs), field-programmable gate arrays (FPGAs), and the like.

The communication unit 52 includes a communication module and has a function of communicating with the data server 60 via the communication network 1.

The display panel 54 can include a liquid crystal panel, an organic electro luminescence (EL) display, or the like.

The operation unit 55 includes, for example, a keyboard, a mouse, and the like, and can operate an icon, and the like, displayed on the display panel 54, move and operate a cursor, input characters, and the like. The operation unit 55 may include a touch panel.

The storage unit 56 can include, for example, a hard disk, a semiconductor memory, or the like, and stores a computer program 57 (program product), a drug library 58, and necessary information (for example, a processing result obtained by the medical pump setting device 50 and the like).

The computer program 57 is an application program for setting the cooperative administration function of the medical pump, and specifically, is an application program (library manager) for editing a drug library. The computer program 57 may be downloaded from an external device via the communication unit 52 and stored in the storage unit 56. The computer program 57 recorded on a recording medium (for example, an optically readable disk storage medium such as a CD-ROM) may be read by a recording medium reading unit and stored in the storage unit 56. The computer program 57 can be deployed so as to be executable on a single computer or on a plurality of computers disposed at a single site or distributed over a plurality of sites and interconnected by a communication network. The computer program 57 may be operable in a state where the communication network is disconnected once after being set operable.

The memory 53 may include a semiconductor memory, such as a static random access memory (SRAM), a dynamic random access memory (DRAM), or a flash memory. The control unit 51 can execute the computer program 57, the computer program 57 being deployed in the memory 53. The control unit 51 may execute processing defined by the computer program 57. That is, the processing executed by the control unit 51 corresponds to processing executed in accordance with the computer program 57.

The drug library 58 is obtained by acquiring a required drug library from the drug library DB 61 storing a plurality of drug libraries via the communication unit 52 and storing the acquired drug library in the storage unit 56. The drug library 58 includes both the drug library acquired from the drug library DB 61 and a drug library edited by the medical pump setting device 50.

The drug library 58 stores setting parameters for implementing the cooperative administration function of the medical pump such as the syringe pump 100. The drug library 58 includes administration information for each drug. Specifically, examples of the administration information include setting information such as a reference flow rate, upper and lower limit values of a flow rate, a drug code, and a drug color for each of thousands of types of drugs. A collection of a plurality of pieces of setting information for one drug is also referred to as a "drug profile". By using the drug library 58, a medical practitioner does not need to perform complicated administration setting each time and easily performs selection of a drug and setting of the drug, and for example, erroneous administration due to setting errors in, for example, a flow rate (mL/h), a dose (mL), and the like can be prevented.

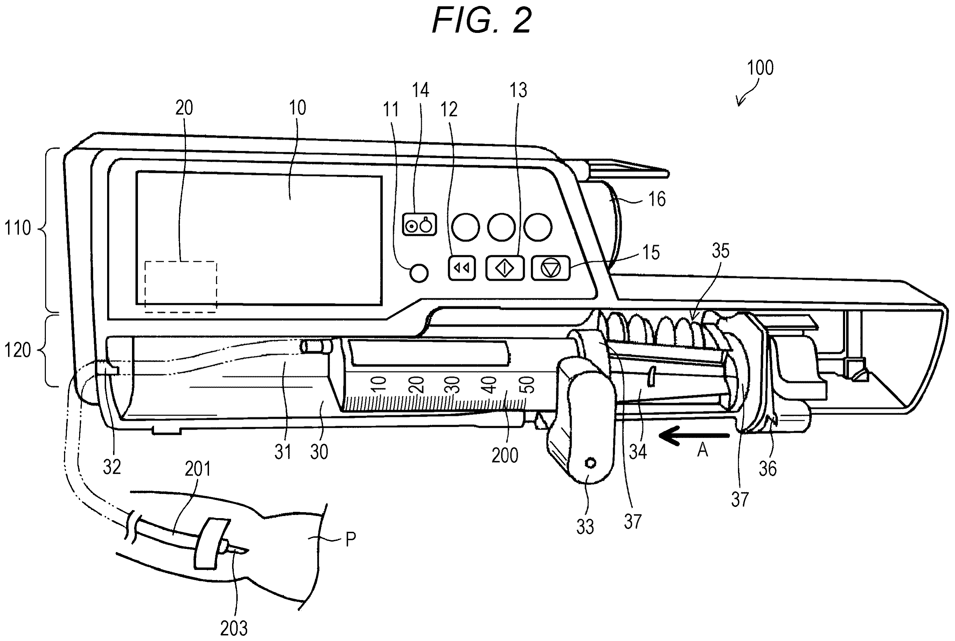

Fig. 2 is a view illustrating an example of a configuration of the syringe pump 100. A main body of the syringe pump 100 has a main body upper portion 110 provided with a display unit 10 and a main body lower portion 120 accommodating a syringe 200. The main body lower portion 120 includes a placement portion 30 on which the syringe 200 is placed, an accommodating portion 31 which is a recess having a semicircular cross section and accommodating the syringe 200, a fixing portion 32 which is provided at an end of the accommodating portion 31 and detachably clamp a tube 201, a clamp 33 for fixing the syringe 200 placed on the placement portion 30, a slider 36 and a motor (not illustrated) for fixing a plunger flange 37 provided on the syringe 200, a syringe plunger drive unit 35 for pushing the syringe plunger 34 provided on the syringe 200, and the like.

A controller 20 is provided in the main body upper portion 110. The controller 20 controls the operation of the syringe pump 100. The controller 20 includes a microcomputer (microprocessor), a read only memory (ROM), a random access memory (RAM), a non-volatile memory, a clock, and the like. The clock is used for acquisition of a current time, measurement of an elapsed time of a predetermined feeding operation, measurement of a reference time of feeding speed control, and the like.

The main body upper portion 110 is provided with an operation unit including various operation buttons and an indicator lamp. The operation unit includes, for example, an operation indicator 11 indicating an operation state of the syringe pump 100, a fast delivery switch button 12, a start switch button 13, a power on/off button 14, a stop switch button 15, and the like. The operation unit may include other operation buttons. At an end of the main body upper portion 110, a setting dial 16 for setting a flow rate and a dose of a drug, a weight of a patient, and the like is provided.

The power on/off button 14 is used to turn on and off the power of the syringe pump 100. The fast delivery switch button 12 is used to fill the tube connected to the syringe 200 and a tip of the indwelling needle 203 with the drug. The start switch button 13 is used to start feeding. The stop switch button 15 is used to temporarily stop the feeding in the middle.

As a method of setting a flow rate of feeding and the like, there are various modes such as a mL/h mode, a µg/kg/min mode, a mg/kg/h mode, and a drug library mode. In a case where the medical practitioner uses the drug library mode, previously registered drug and administration method are selected, and a dose and the like corresponding to the selected drug and administration method are set using the setting dial 16.

The syringe pump 100 continuously feeds a liquid medicine in the syringe at a set flow rate (mL/h). The controller 20 controls the feeding by controlling a motor rotation signal based on setting information set for each drug. The syringe plunger drive unit 35 (motor) can perform the feeding by pushing the syringe plunger 34 in a direction of reference sign A in Fig. 2 via the slider 36 or the like. As a result, the medical practitioner can inject the drug in the syringe 200 into a patient P through the tube 201 and the indwelling needle 203.

Next, the cooperative administration operation of the syringe pumps 100 by the cooperative administration function will be described.

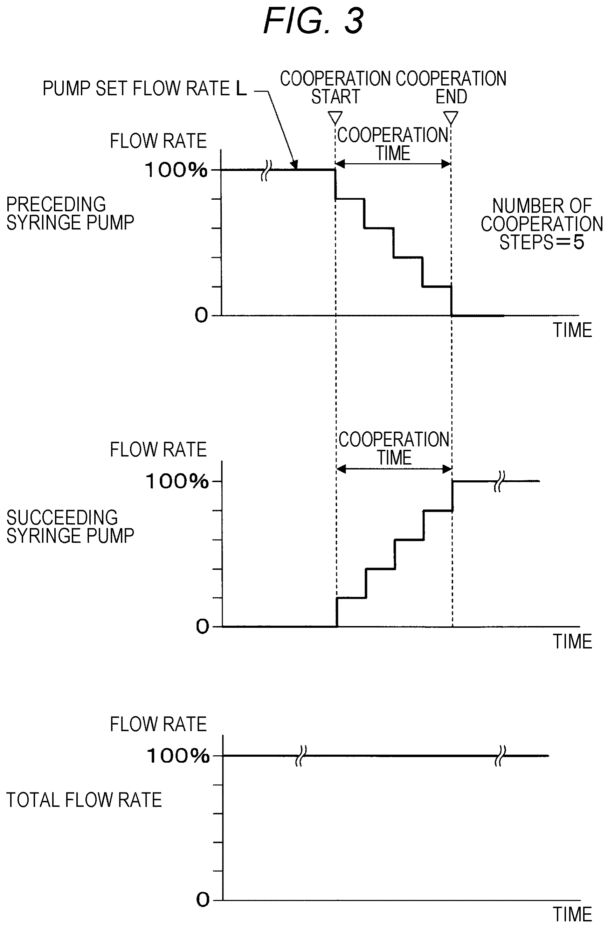

Fig. 3 is a view illustrating an example of the cooperative administration operation of the syringe pumps 100. The cooperative administration operation illustrated in Fig. 3 implements a method (referred to as "W method") of continuously administering the same type of drug by causing the two syringe pumps 100 (a preceding syringe pump and a succeeding syringe pump) to cooperate. The medical pump setting device 50 can perform control such that administration of a drug is not interrupted for, for example, a disease that affects a living body of a patient when the administration of the drug is interrupted even for a moment or a patient after surgery. In the cooperative administration operation, feeding of the second syringe pump 100 (succeeding pump) is started not after completion of feeding of the first syringe pump 100 (preceding syringe pump), but shortly before the completion.

The preceding syringe pump feeds the drug at a pump set flow rate L (also referred to as "pre-cooperation flow rate") corresponding to a flow rate of 100%. The medical pump setting device 50 decreases the flow rate of the preceding syringe pump at the time of start of cooperation and starts the feeding of the succeeding syringe pump. At the time of end of the cooperation, the flow rate of the preceding syringe pump becomes 0%, and a flow rate of the succeeding syringe pump becomes 100% (the pump set flow rate L). A period of time from the cooperation start time to the cooperation end time is referred to as a cooperation time.

Within the cooperation time, the flow rate of the preceding syringe pump decreases stepwise, and the flow rate of the succeeding syringe pump increases stepwise. In the feeding by the W method, the medical pump setting device 50 controls a total flow rate of the flow rate of the preceding syringe pump and the flow rate of the succeeding syringe pump to be the pump set flow rate L. According to the medical pump setting device 50 of the present embodiment, a cooperation step time and a flow rate in each cooperation step can be determined by setting the cooperation time and the number of cooperation steps (in the example of Fig. 3, the cooperation step = 5).

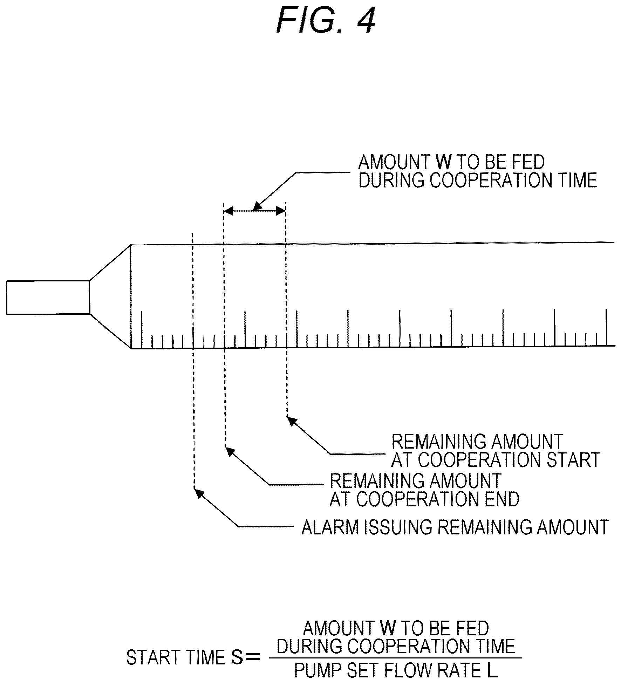

Fig. 4 is a view illustrating an example of a state of the preceding syringe pump during the cooperative administration operation. The medical pump setting device 50 starts the cooperative administration operation when a remaining amount of the drug of the preceding syringe pump performing feeding at the pump set flow rate L decreases to some extent. As illustrated in Fig. 4, an amount obtained by subtracting the remaining amount of the drug at the cooperation end from a remaining amount of the drug at the cooperation start is an amount W to be fed during the cooperation time. In order for the medical pump setting device 50 to normally end the cooperative administration operation, the remaining amount of the drug of the preceding syringe pump at the cooperation end needs to be larger than an alarm issuing remaining amount. If the remaining amount of the drug of the preceding syringe pump becomes equal to or smaller than the alarm issuing remaining amount during the cooperative administration operation, the cooperative administration operation is stopped, and the succeeding syringe pump continues operating at the pump set flow rate L.

Therefore, during the cooperative administration operation (specifically, until the continuous administration operation is completed), the medical pump setting device 50 needs to calculate a start time S for determining when to start the cooperative administration operation in order to prevent the remaining amount of the drug of the preceding syringe pump from being equal to or smaller than the alarm issuing remaining amount. The start time S is a period of time required for feeding the amount W to be fed during the cooperation time at the pump set flow rate L, and can be calculated by a formula of Start time S = Amount W to be fed during cooperation time/Pump set flow rate L. Since the amount W to be fed during the cooperation time depends on the number of cooperation steps, the cooperation step time, and the flow rate in each cooperation step, the start time S needs to be calculated based on the pump set flow rate L, the number of cooperation steps, the cooperation step time, and the flow rate in each cooperation step. Typically, it is difficult for the medical practitioner to determine which value to set for each of the pump set flow rate L, the number of cooperation steps, the cooperation step time, the flow rate in each cooperation step, and the like.

The medical pump setting device 50 of the present embodiment can calculate and display the start time S by receiving setting parameters for setting the start time S of cooperative administration for preventing a remaining infusion amount during the cooperative administration operation of causing a plurality of medical pumps to cooperate from being equal to or smaller than an alarm value. Hereinafter, an example in which the control unit 51 of the medical pump setting device 50 calculates the start time S will be described.

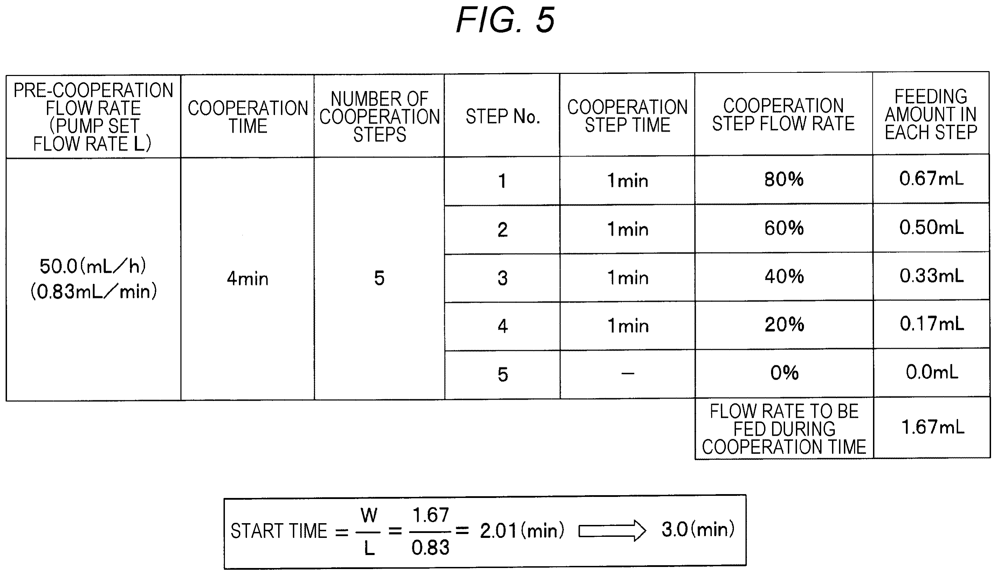

Fig. 5 is a view illustrating an example of a case where the control unit 51 calculates the start time S. In the example of Fig. 5, setting parameters for setting the start time S include the pre-cooperation flow rate (pump set flow rate) L, the cooperation time, and the number of cooperation steps. When receiving the setting parameters, the control unit 51 determines the cooperation step time and the cooperation step flow rate based on the received cooperation time (for example, 4 min) and the received number of cooperation steps (for example, 5). The cooperation step time is a time length of each cooperation step, and the cooperation step time is set to 1 min in order to divide the cooperation time 4 min into five sections. In the example of Fig. 5, since the number of cooperation steps is five, flow rates in cooperation step Nos. 1 to 5 are 80%, 60%, 40%, 20%, and 0%, respectively.

Furthermore, the control unit 51 calculates the flow rate in each step and a feeding amount in each step to calculate the amount W to be fed during the cooperation time. In the example of Fig. 5, the control unit 51 sets the flow rate in step No. 1 as 80% of the pre-cooperation flow rate 50.0 mL/h, and calculates the feeding amount for 1 min as 0.67 mL. Similarly, the flow rate in step No. 2 is 60% of the pre-cooperation flow rate of 50.0 mL/h, the feeding amount for 1 min is 0.50 mL. The flow rate in step No. 3 is 40% of the pre-cooperation flow rate of 50.0 mL/h, and the feeding amount for 1 min is 0.33 mL. The flow rate in step No. 4 is 20% of the pre-cooperation flow rate of 50.0 mL/h, and the feeding amount for 1 min is 0.17 mL. The flow rate in step No. 5 is 0% of the pre-cooperation flow rate of 50.0 mL/h, and the feeding amount for 1 min is 0.0 mL. The amount W to be fed during the cooperation time is 1.67 mL. The start time S is obtained as the amount W to be fed during the cooperation time/the pump set flow rate L = 1.67/0.83 = 2.01 (min). Since the start time S is set to an integer value for convenience, the start time S is 3.0 (min).

The medical pump equipped with the cooperative administration function has a callback function as a related function. The callback function is a function of issuing a start cooperation miss alarm to the succeeding syringe pump side at the start of cooperative administration, and starting cooperative administration by operating the start switch button 13. In a case where the callback function is set to OFF, the medical pump automatically starts cooperative administration when a set start time passes without issuing the cooperation start miss alarm. In a case where the callback function is set to ON, a margin time of about one minute to several minutes may be added to a start time to be set in consideration of the fact that some time is required until the medical practitioner such as a nurse or a doctor operates the start switch button 13 after the cooperation start miss alarm is issued.

Next, a method of setting the medical pump by the medical pump setting device 50 will be described.

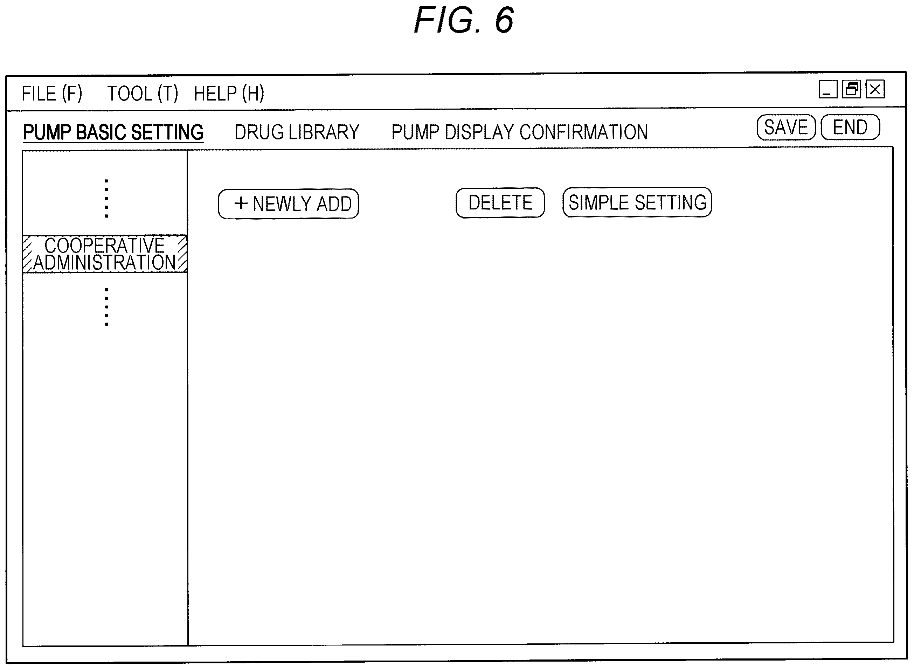

Fig. 6 is a view illustrating an example of a pump basic setting screen. Various screens illustrated in Fig. 6 and subsequent drawings correspond to an editing screen of a drug library for setting the medical pump. On the pump basic setting screen, the medical practitioner selects a "cooperative administration" icon, operates a "+ newly add" icon, and operates a "simple setting" icon to display a cooperative administration simple setting screen (also referred to as a "first cooperative administration screen") to receive setting parameters for setting a start time of cooperative administration on the display panel 54.

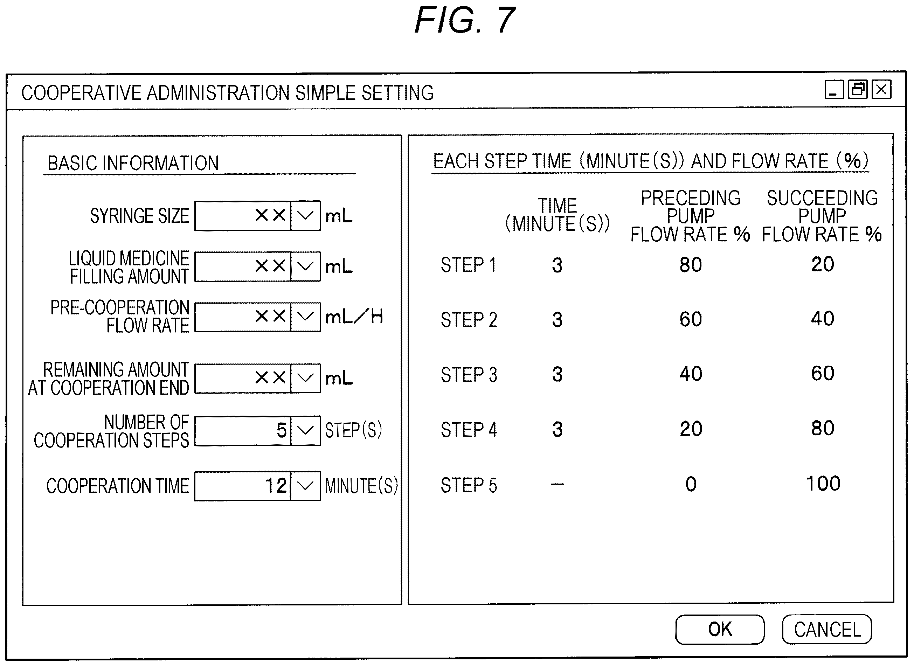

Fig. 7 is a view illustrating a first example of the cooperative administration simple setting screen. The cooperative administration simple setting screen displays a "basic information" field for receiving input or selection of the setting parameters on the display panel 54. The setting parameters include, regarding the preceding syringe pump, a syringe size, a liquid medicine filling amount in the syringe 200, the pre-cooperation flow rate (pump set flow rate L), the remaining amount at the cooperation end, the number of cooperation steps (5 in the example of Fig. 7), and the cooperation time (12 minutes in the example of Fig. 7).

When the setting parameters are input or selected on the cooperative administration simple setting screen, the control unit 51 calculates a time in each cooperation step (cooperation step time) and flow rates of the preceding syringe pump and the succeeding syringe pump in each cooperation step (ratios with respect to the pump set flow rate L when the pump set flow rate L is set to 100%) based on the number of cooperation steps and the cooperation time, and displays the calculated values in the cooperative administration simple setting screen. Since the control unit 51 performs control such that a total flow rate of the flow rate of the preceding syringe pump and the flow rate of the succeeding syringe pump in each cooperation step coincides with the pump set flow rate L, the sum of the flow rate of the preceding syringe pump and the flow rate of the succeeding syringe pump is determined to be 100%.

In the example of Fig. 7, since the cooperation time is 12 minutes and the number of cooperation steps is five, the time in each of Cooperation steps 1 to 4 is 3 minutes, the flow rates of the preceding syringe pump decrease stepwise such as 80%, 60%, 40%, 20%, and 0%, and the flow rates of the succeeding syringe pump increase stepwise such as 20%, 40%, 60%, 80%, and 100%. When the medical practitioner operates an "OK" icon, the control unit 51 displays a cooperative administration screen (also referred to as a "second cooperative administration screen") including a start time based on the input or selected setting parameters. When the medical practitioner operates a "cancel" icon, the input or selected setting parameters are canceled, and the medical practitioner can input or select the setting parameters again.

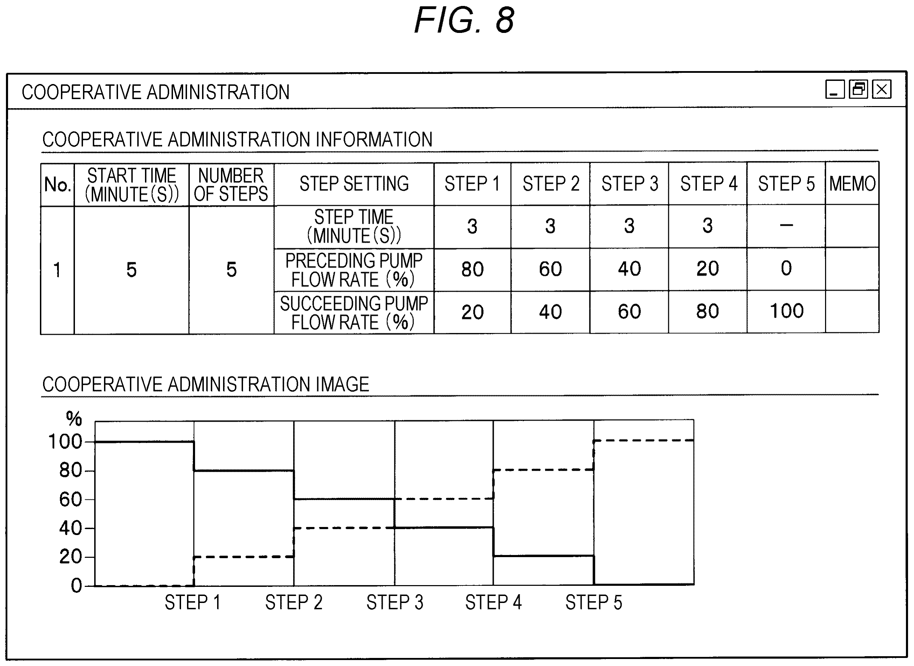

Fig. 8 is a view illustrating a first example of the cooperative administration screen. Cooperative administration information and a cooperative administration image are displayed on the cooperative administration screen. Examples of the cooperative administration information include a start time and the number of steps, and as step settings in each step, a step time, a preceding syringe pump flow rate, and a succeeding syringe pump flow rate. The control unit 51 displays the step settings as the same numerical values as "each step time (minute(s)) and flow rate (%)" illustrated in Fig. 7. Note that the start time is displayed in the cooperative administration screen in the example of Fig. 8, but a display example of the start time is not limited to the example of Fig. 8, and the control unit 51 may display the start time on a pop-up screen or the like different from the cooperative administration screen.

The control unit 51 displays the cooperative administration image as a graph such that the cooperative administration operation of the preceding syringe pump and the succeeding syringe pump can be visually and intuitively understood. In Figs. 8, 9, and 10, a solid-line graph illustrates the transition of the flow rate of the preceding syringe pump for each cooperation step, and a broken-line graph illustrates the transition of the flow rate of the succeeding syringe pump for each cooperation step. As a result, the medical practitioner can compare and confirm the cooperative administration operation of each of the preceding syringe pump and the succeeding syringe pump, and can easily grasp an operation image of the syringe pumps based on the set content.

As described above, the control unit 51 displays the cooperative administration simple setting screen (first cooperative administration screen) to receive input or selection of the setting parameters for setting the start time of cooperative administration for preventing the remaining infusion amount during the cooperative administration operation of causing the plurality of medical pumps to cooperate from being equal to or smaller than the alarm value, and displays the start time when the setting parameters are input or selected on the cooperative administration simple setting screen.

The setting parameters include the syringe size of the preceding syringe pump, the pre-cooperation flow rate (pump set flow rate), the remaining amount at cooperation completion, the number of cooperation steps, and the cooperation time.

With the configuration of the present embodiment described above, the medical practitioner can normally end the cooperative administration operation (W method) only by inputting or selecting the setting parameters, and can easily set the cooperative administration function of the medical pump. According to the present embodiment, the medical practitioner does not need to individually calculate and set many setting items (for example, the step time, the step flow rates (preceding syringe pump flow rate and succeeding syringe pump flow rate), and the like in addition to the start time) for the cooperative administration function, and the cooperative administration function of the medical pump can be easily set.

The control unit 51 may adjust the start time based on the remaining amount at the cooperation end input or selected by the medical practitioner. For example, if the start time is short (start timing is set to be late), a remaining amount of the liquid medicine in the preceding syringe pump at a time point when the cooperative administration operation ends tends to decrease, and thus it is sufficient to adjust the start time such that the remaining amount of the liquid medicine in the preceding syringe pump at the time point when the cooperative administration operation ends becomes the remaining amount at the cooperation end. As a result, the remaining amount of the liquid medicine in the preceding syringe pump at the end of the cooperative administration operation can be adjusted only by setting the remaining amount at the cooperation end, so that the waste liquid treatment can be reduced.

When the setting parameters are input or selected on the cooperative administration simple setting screen, the control unit 51 may display the cooperative administration screen (second cooperative administration screen) including the number of cooperation steps, the cooperation step time, and the flow rate in each cooperation step of each of the plurality of medical pumps (the preceding syringe pump and the succeeding syringe pump). As a result, the medical practitioner does not need to calculate the cooperation step time, the flow rate in each cooperation step, and the like, so that the cooperative administration function of the medical pump can be easily set.

When the setting parameters are input or selected on the cooperative administration simple setting screen, the control unit 51 may display the cooperative administration screen (second cooperative administration screen) including a cooperation image indicating the transition of the flow rate of each of the plurality of medical pumps (the preceding syringe pump and the succeeding syringe pump). As a result, the medical practitioner can visually confirm the operation image of the cooperative administration (W method).

The setting of the cooperative administration operation needs to be changed when usage and dosage of a drug based on the cooperative administration image of the cooperative administration screen illustrated in Fig. 8 do not match usage and dosage of a drug used in the cooperative administration (W method) or does not match usage and dosage according to an instruction of a doctor, or when usage and dosage of a drug needs to be changed according to the instruction of the doctor. Hereinafter, a countermeasure in such a case will be described.

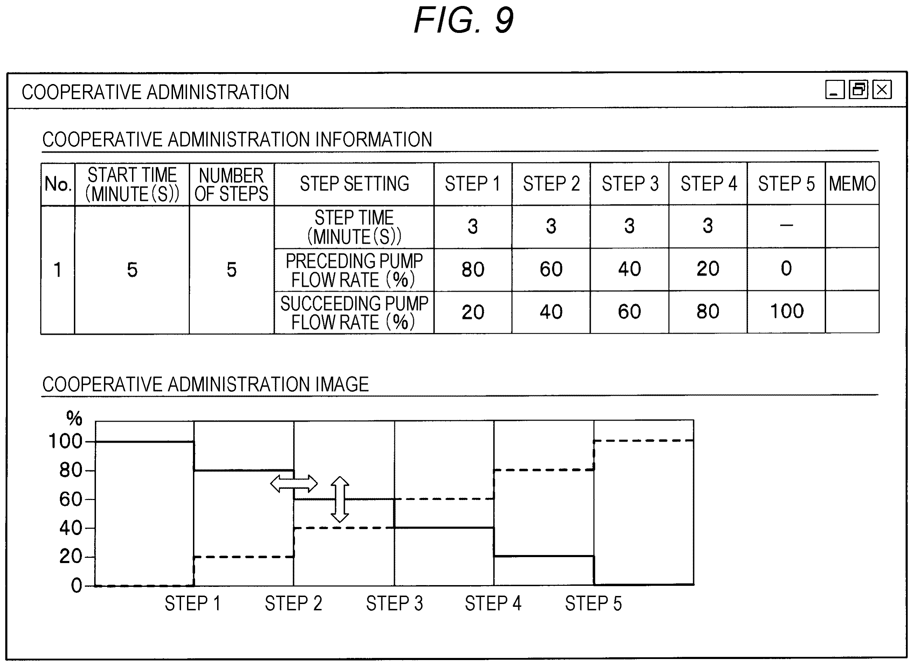

Fig. 9 is a view illustrating a second example of the cooperative administration screen. In the second example, the control unit 51 can adjust the transition of the flow rate of the preceding syringe pump in the cooperative administration image after receiving an operation (for example, drag operation using a mouse) on the graph illustrated for each cooperation step. In the example of Fig. 9, the control unit 51 can receive an adjustment operation of lengthening or shortening a required cooperation step time (double-headed arrow) and an adjustment operation of increasing or decreasing a flow rate (%) in a required cooperation step (double-headed arrow). Locations for receiving the operations and the number of operations are not limited to those in the example of Fig. 9.

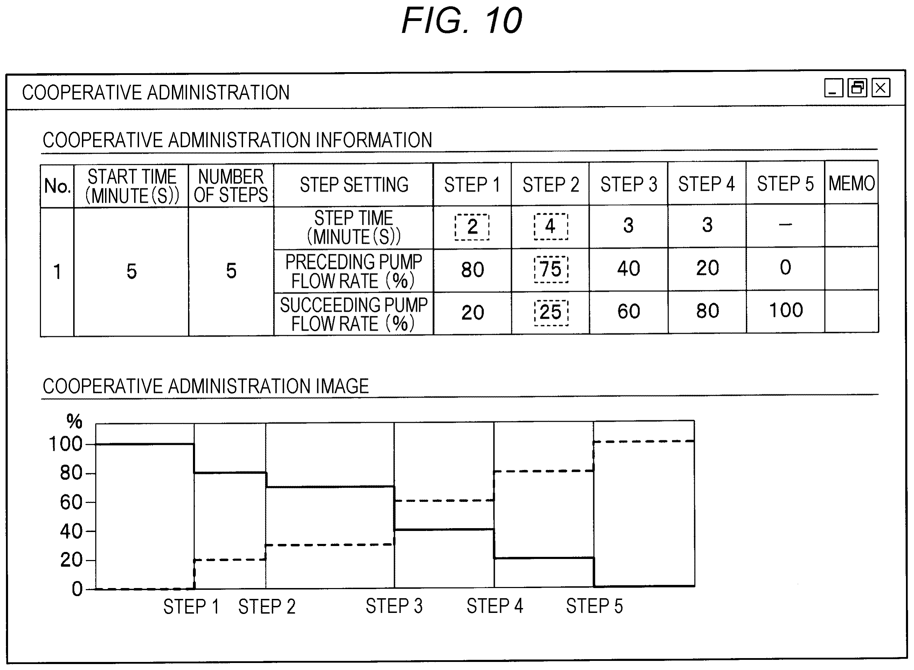

Fig. 10 is a view illustrating a third example of the cooperative administration screen. The third example illustrates a state in which the control unit 51 corrects the transition of the flow rate of the preceding syringe pump after receiving the operation on the illustrated graph for each cooperation step. As illustrated in Fig. 10, the operation on the graph causes the control unit 51 to change the step time in step 1 from 3 minutes to 2 minutes and to change the step time in step 2 from 3 minutes to 4 minutes. The control unit 51 changes the preceding syringe pump flow rate in step 2 from 60% to 75%, and changes the succeeding syringe pump flow rate in step 2 from 40% to 25%.

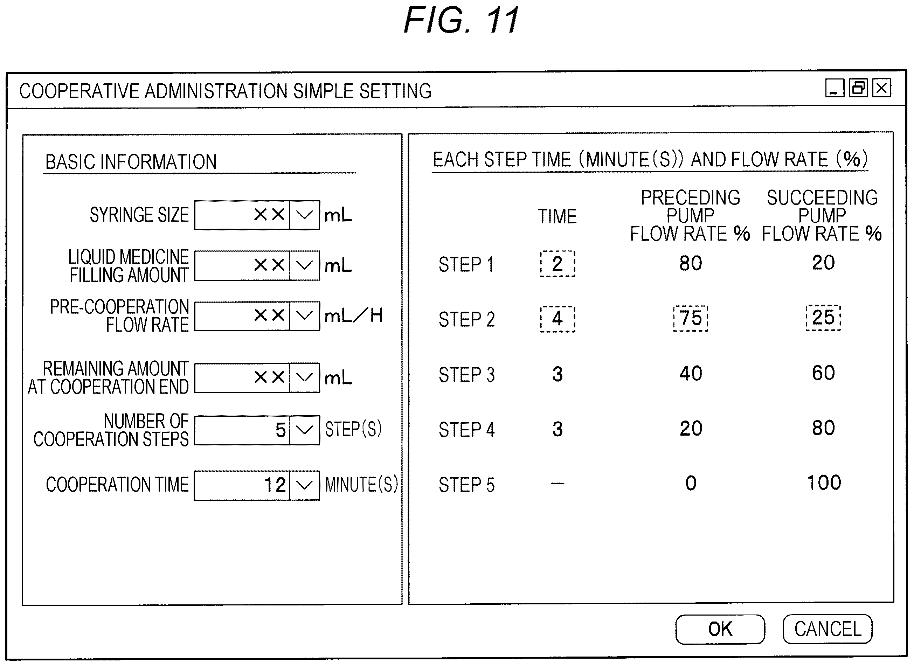

Fig. 11 is a view illustrating a second example of the cooperative administration simple setting screen. The second example illustrates a state in which the control unit 51 corrects the transition of the flow rate of the preceding syringe pump after receiving the operation on the illustrated graph for each cooperation step. As illustrated in Fig. 11, the operation on the graph causes the control unit 51 to change the step time in step 1 from 3 minutes to 2 minutes and to change the step time in step 2 from 3 minutes to 4 minutes. The preceding syringe pump flow rate in step 2 is changed from 60% to 75%, and the succeeding syringe pump flow rate in step 2 is changed from 40% to 25%.

As described above, the control unit 51 may receive the operation on the cooperation image indicating the transition of the flow rate of each of the plurality of medical pumps in the cooperative administration screen, and adjust and display at least one of the cooperation step time and the flow rate in each cooperation step of each of the plurality of medical pumps displayed on the cooperative administration simple setting screen in accordance with the received operation. As a result, the medical practitioner can change each of the setting parameters from a cooperative administration operation image, and for example, can adjust the usage and dosage of the drug used in the cooperative administration (W method) while visually confirming the usage and dosage from the operation image.

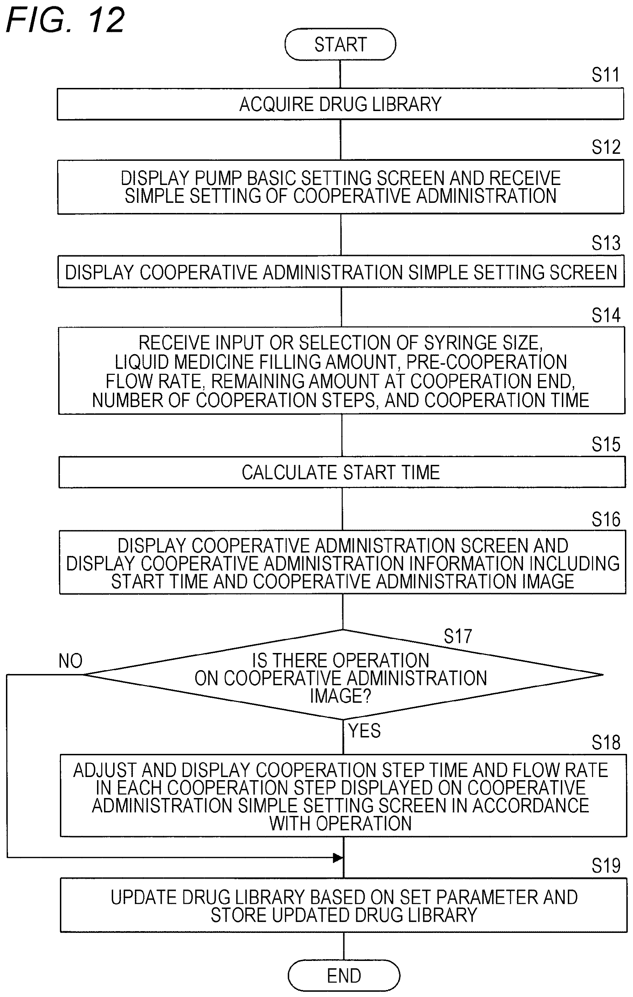

Fig. 12 is a view illustrating an example of a processing procedure of the medical pump setting device 50. The control unit 51 acquires a drug library from the data server 60 (S11), displays the pump basic setting screen on the display panel 54, and receives simple setting of cooperative administration (S12). The control unit 51 displays the cooperative administration simple setting screen (S13), and receives input or selection of a syringe size of a preceding syringe pump, a liquid medicine filling amount, a pre-cooperation flow rate, a remaining amount at the cooperation end, the number of cooperation steps, and a cooperation time (S14).

The control unit 51 calculates a start time (S15). An example of the calculation of the start time is described in Figs. 4 and 5. The control unit 51 displays the cooperative administration screen, and displays cooperative administration information including the start time and a cooperative administration image (S16). Examples of the cooperative administration information and the cooperative administration image are given in Fig. 8. The control unit 51 determines whether there is an operation on the cooperative administration image (S17). An example of the operation on the cooperative administration image is given in Fig. 9.

When there is an operation on the cooperative administration image (YES in S17), the control unit 51 adjusts and displays a cooperation step time displayed on the cooperative administration simple setting screen and a flow rate in each cooperation step in accordance with the operation (S18), updates the drug library based on such setting parameters and stores the updated drug library in the storage unit 56 (S19), and ends the processing. When there is no operation on the cooperative administration image (NO in S17), the control unit 51 performs the processing of step S19.

### Reference Signs List

- 1: Communication network
- 50: Medical pump setting device
- 51: Control unit
- 52: Communication unit
- 53: Memory
- 54: Display panel
- 55: Operation unit
- 56: Storage unit
- 57: Computer program
- 58: Drug library
- 60: Data server
- 61: Drug library DB
- 100: Syringe pump
- 200: Syringe

## Claims

1. A computer program for causing a computer to execute processing of:
displaying a first cooperative administration screen to receive setting parameters for setting a start time of cooperative administration for preventing a remaining infusion amount during a cooperative administration operation of causing a plurality of medical pumps to cooperate from being equal to or smaller than an alarm value; and
displaying the start time when the setting parameters are input or selected on the first cooperative administration screen.

2. The computer program according to claim 1, for causing the computer to execute processing of
displaying a second cooperative administration screen including a number of cooperation steps, a cooperation step time, and a flow rate in each of the cooperation steps of each of the plurality of medical pumps when the setting parameters are input or selected on the first cooperative administration screen.

3. The computer program according to claim 1, for causing the computer to execute processing of
displaying a second cooperative administration screen including a cooperation image indicating transition of a flow rate of each of the plurality of medical pumps when the setting parameters are input or selected on the first cooperative administration screen.

4. The computer program according to claim 3, for causing the computer to execute processing of:
receiving an operation on the cooperation image indicating the transition of the flow rate of each of the plurality of medical pumps in the second cooperative administration screen; and
adjusting and displaying at least one of a cooperation step time and the flow rate in each cooperation step of each of the plurality of medical pumps displayed on the first cooperative administration screen in accordance with the received operation.

5. The computer program according to any one of claims 1 to 4, wherein
the plurality of medical pumps include a preceding medical pump that gradually decreases a flow rate during the cooperative administration operation, and
the start time is a period of time required when an amount to be fed from the preceding medical pump in the cooperative administration is fed at a set flow rate of the preceding medical pump.

6. The computer program according to any one of claims 1 to 4, wherein
the plurality of medical pumps include a preceding medical pump that gradually decreases a flow rate during the cooperative administration operation, and
the setting parameters include a syringe size of the preceding medical pump, a set flow rate, a remaining amount at cooperation completion, a number of cooperation steps, and a cooperation time.

7. A medical pump setting device comprising a control unit,
wherein the control unit
displays a first cooperative administration screen to receive a setting parameter for setting a start time of cooperative administration for preventing a remaining infusion amount during a cooperative administration operation of causing a plurality of medical pumps to cooperate from being equal to or smaller than an alarm value, and
displays the start time when the setting parameter is input or selected on the first cooperative administration screen.

8. A medical pump setting system comprising:
the medical pump setting device according to claim 7; and
a plurality of medical pumps.

9. A medical pump setting method comprising:
displaying a first cooperative administration screen to receive a setting parameter for setting a start time of cooperative administration for preventing a remaining infusion amount during a cooperative administration operation of causing a plurality of medical pumps to cooperate from being equal to or smaller than an alarm value; and
displaying the start time when the setting parameter is input or selected on the first cooperative administration screen.
